# FASCICULE DE BREVET EUROPEEN

(11) **EP 1 736 192 B1**
(45) Date de publication et mention de la délivrance du brevet: **26.03.2008**
(21) Numéro de dépôt: 06290984.1
(22) Date de dépôt: 16.06.2006
(51) Int. Cl.: A61M 5/32

(54) **Aiguille d'injection perforante**
Perforierende Injektionsnadel
Perforating injection needle

(30) Priorité: 20.06.2005 FR 0506215
(43) Date de publication de la demande: 27.12.2006
(73) Titulaire: Villette, Alain, 79700 Saint Pierre Les Echaubrognes (FR)
(72) Inventeur: Villette, Alain, 79700 Saint Pierre Les Echaubrognes (FR)
(74) Mandataire: Delaveau, Sophie

(56) Documents cités:
- FR-A- 2 481 930
- GB-A- 748 451
- US-A- 3 308 822
- US-A- 4 490 139
- US-A- 5 820 609

## Description

L'invention concerne une aiguille d'injection perforante pour l'injection d'un produit pharmaceutique dans un tissu dur du corps humain ou animal et pour une injection moins douloureuse lors d'une injection dans un tissu mou.

Lors de soins dentaires, il est nécessaire, d'administrer au patient une anesthésie locale. Cette injection est habituellement faite dans les gencives et elle produit des effets dans les tissus mous périphériques. Indépendamment des désagréments que cela représente pour le patient, l'efficacité de l'anesthésie n'est pas toujours optimale. Cela est essentiellement dû au fait que le produit anesthésique se répand partiellement à distance de la dent à traiter au lieu d'y rester concentré.

Lors de soins dermatologiques chez l'homme ou chez l'animal, il est parfois aussi utile de disposer d'une aiguille pour effectuer une injection de manière moins douloureuse ou de manière plus sûre.

Sur certaines aiguilles, pour que le praticien puisse travailler avec précision, l'embase de l'aiguille, partie pouvant être réalisée en plastique ou en métal et permettant de visser l'aiguille sur la seringue, comporte un repère indiquant l'emplacement du biseau principal de l'aiguille. Habituellement, le repère se trouve du côté du biseau, ce qui signifie que, lorsque l'on regarde l'aiguille en vue latérale, on voit à la fois le repère et le biseau principal.

Toutefois, ce positionnement du repère ne permet pas au praticien d'utiliser le biseau correctement, car pour une injection sans douleur le biseau principal doit se situer à plat sur la muqueuse ou la peau. Le praticien doit donc veiller à ce que l'aiguille soit toujours tournée de manière à ce qu'il ne voit pas le repère, ce qui ne correspond pas à un positionnement précis, mais à un positionnement très approximatif.

Pour remédier aux différents inconvénients énoncés ci avant, des aiguilles d'injection perforantes ont été développées afin de rendre possible, par exemple au dentiste, de pénétrer à l'intérieur de la masse osseuse là où se situent les dents et d'y déposer le produit anesthésique. Ainsi, celui-ci est déposé dans l'os spongieux interdentaire et on obtient une anesthésie immédiate des dents à traiter. L'os étant bordé par une corticale, c'est-à-dire par une partie dure, formant une boîte, le liquide anesthétique est moins exposé au phénomène de migration que ce n'est le cas dans un tissu périphérique irrigué par le sang. Au dermatologue et au vétérinaire, les aiguilles à biseau plus incisant permettent de pénétrer la ou les couches superficielles de la peau sans douleur.

Les aiguilles d'injection perforantes ont été développées à partir d'aiguilles d'injection habituellement utilisées. Ainsi, ces aiguilles d'injection perforantes comprennent un corps tubulaire ayant deux extrémités opposées, dont une première extrémité pourvue d'un biseau unique court (postérieur) est destinée à être reliée à des moyens d'entraînement en rotation et à des moyens d'amenée ou d'approvisionnement de liquide. C'est en référence à ce biseau, lors de la fabrication, qu'est positionné le marquage sur l'embase permettant le repérage du biseau antérieur. La seconde extrémité, qui est destinée à pénétrer dans le corps humain ou animal, est pourvue d'un biseau long (antérieur) constituant le biseau principal qui est complété par deux autres biseaux, les biseaux secondaires. Dans la suite de la description, le biseau principal définit la face avant de la seconde extrémité de l'aiguille.

A ce jour, les secondes extrémités des aiguilles avec leurs trois biseaux revêtent principalement l'une ou l'autre de deux formes. Selon la première forme, qui est la plus répandue, les deux biseaux secondaires sont situés sur la surface plane du biseau principal. Ils rendent l'aiguille plus pointue. C'est le biseau « lancette ». Selon la seconde forme, moins répandue que la première, les deux biseaux secondaires sont situés en opposition au biseau principal et forment donc des contre-biseaux. Cette disposition des trois biseaux donne à la pointe de l'aiguille une forme de pyramide. C'est le biseau « back cut ».

Lors de l'utilisation, en rotation, pour une perforation, d'une aiguille ayant l'une ou l'autre des dispositions du triple biseau décrit ci avant, le pouvoir de pénétration dans la masse osseuse est médiocre.

Par ailleurs, de telles aiguilles d'injection ont un angle de pointe de l'ordre de 60 degrés. Le biseau principal a un angle, par rapport à l'axe longitudinal de l'aiguille, de l'ordre de 10 et 15 degrés.

Toutefois, au fil de diverses utilisations pratiques, il s'est avéré que ces aiguilles d'injection écartent les tissus lorsque l'on pratique une injection dans la gencive. La pénétration est donc assez douloureuse. De plus, ces aiguilles forent assez mal du fait que les angles formés par l'intersection des biseaux entre eux ou des biseaux et de la paroi circulaire sont voisins de, ou même supérieurs à, 60 degrés.

Le but de l'invention est de proposer une aiguille d'injection perforante palliant les inconvénients décrits avant.

Le but de l'invention est atteint avec une aiguille d'injection perforante pour l'injection d'un produit pharmaceutique dans un corps humain ou animal, qui comprend un corps tubulaire ayant deux extrémités opposées, à savoir une première extrémité destinée à être reliée à des moyens d'entraînement en rotation et à des moyens d'amenée de produit et destinée aussi à permettre le positionnement correct du repère du biseau antérieur sur l'embase une seconde extrémité destinée à pénétrer dans le corps humain ou animal et pourvue à cet effet d'un biseau principal et d'au moins un biseau secondaire.

Selon l'invention, la seconde extrémité de l'aiguille est pourvue en opposition par rapport au biseau principal d'un seul biseau secondaire formant avec le biseau principal un seul bord incisant et une pointe de l'aiguille.

Le fait que l'aiguille de l'invention est pourvue d'un bord incisant, signifie, notamment par opposition aux aiguilles ayant deux contre-biseaux, que l'aiguille est spécialement conformée pour un travail de perforation et d'incision, travail qui implique en premier une incision pour amorcer un enlèvement de tissu et ensuite une coupe du tissu en lamelles pour effectuer l'enlèvement du tissu, l'incision et la coupe étant réalisées moyennant le bord incisant de l'aiguille qui présente des caractéristiques géométriques particulièrement étudiées pour cette tâche.

En effet, par opposition aux aiguilles à contre-biseaux qui, essentiellement, écartent le tissu dans lequel elles pénètrent, l'aiguille de l'invention incise le tissu dans la direction de la coupe, c'est-à-dire suivant un mouvement en spirale avec une composante circonférentielle importante correspondant à la vitesse de rotation de l'aiguille et une composante axiale beaucoup moins importante correspondant à la vitesse de pénétration - ou vitesse axiale - de l'aiguille. Cette incision du tissu est continuellement effectuée, au fur et à mesure que le bord incisant de l'aiguille progresse, et permet d'enlever le tissu sous la forme de lamelles ainsi découpées.

Cette manière de procéder de l'aiguille de l'invention s'applique aussi bien à un tissu mou, par exemple lors d'un traitement dermatologique, qu'à un tissu dur, par exemple lors d'un traitement dentaire.

Selon un premier mode de réalisation, l'aiguille perforante de l'invention n'a qu'un seul biseau secondaire et un seul bord incisant, comme décrit ci avant.

Selon un second mode de réalisation, l'aiguille perforante de l'invention comprend un premier biseau secondaire et un second biseau secondaire. Le premier biseau secondaire correspond, dans la conception et le rôle à remplir, au biseau secondaire unique du premier mode de réalisation. Il est donc disposé en opposition par rapport au biseau principal et forme avec celui-ci un bord incisant. Le second biseau secondaire est disposé en opposition au premier biseau secondaire et adjacent au biseau principal, sur un plan incliné par rapport au biseau principal, et forme avec le biseau principal une arête et, avec le pourtour du corps tubulaire, un bord auxiliaire de coupe dont l'angle varie tout au long du bord de l'aiguille. Le biseau principal et les deux biseaux secondaires forment ensemble une pointe de l'aiguille sous la forme d'une lame plate.

L'aiguille selon le second mode de réalisation peut avoir en outre l'une au moins des caractéristiques ci-après, considérées isolément ou selon toute combinaison techniquement possible :
- le second biseau secondaire est incliné par rapport au biseau principal d'un angle d'inclinaison de l'ordre de 35 degrés ;
- le bord incisant et le bord auxiliaire de coupe enferment entre eux un angle de pointe de l'ordre de 70 à 80 degrés ;
- le biseau principal forme avec un axe longitudinal du corps tubulaire un angle de l'ordre de 10 à 20 degrés ;
- l'embase de l'aiguille est pourvue d'un marquage de positionnement disposé, par rapport à l'axe longitudinal de l'aiguille, opposé au biseau principal.

Grâce à la disposition asymétrique des biseaux secondaires et plus particulièrement grâce au fait que l'aiguille d'injection perforante de l'invention n'a qu'un seul bord incisant, l'aiguille est plus performante dans la perforation et incise les tissus mous, ce qui la rend moins douloureuse. D'une façon générale l'extrémité d'une aiguille est toujours formée par l'intersection d'un plan et d'un tube selon un certain angle et par l'intersection d'un ou deux autres plans qui peuvent tourner autour de l'axe du tube et former avec le premier plan une arête d'angle constant (bord incisant ou coupant) et avec le bord du tube une arête dont l'angle varie.

Un calcul mathématique permet en fonction des angles de rotation des différents plans de calculer les angles formés par les intersections et d'adapter ainsi l'aiguille à la fonction que l'on veut privilégier.

D'autres caractéristiques et avantages de la présente invention ressortiront de la description ci-après de deux modes de réalisation, description faite en référence aux dessins.

Dans les dessins,
la figure 1 montre une aiguille perforante selon l'invention en une vue latérale,
la figure 2 montre une aiguille perforante selon un premier mode de réalisation de l'invention en une vue sur le biseau principal de l'aiguille,
la figure 3 montre une coupe transversale de l'aiguille perforante de la figure 2,
les figures 4A et 4B montrent une aiguille perforante selon un second mode de réalisation de l'invention et une variante en une vue sur le biseau principal de l'aiguille,
la figure 5 montre la face opposée au biseau principal de l'aiguille de la figure 4,
les figures 6A et 6B montrent une coupe transversale de l'aiguille perforante de la figure 4A et de la figure 4B, et
la figure 7 montre une aiguille selon l'invention avec un support portant un marquage.

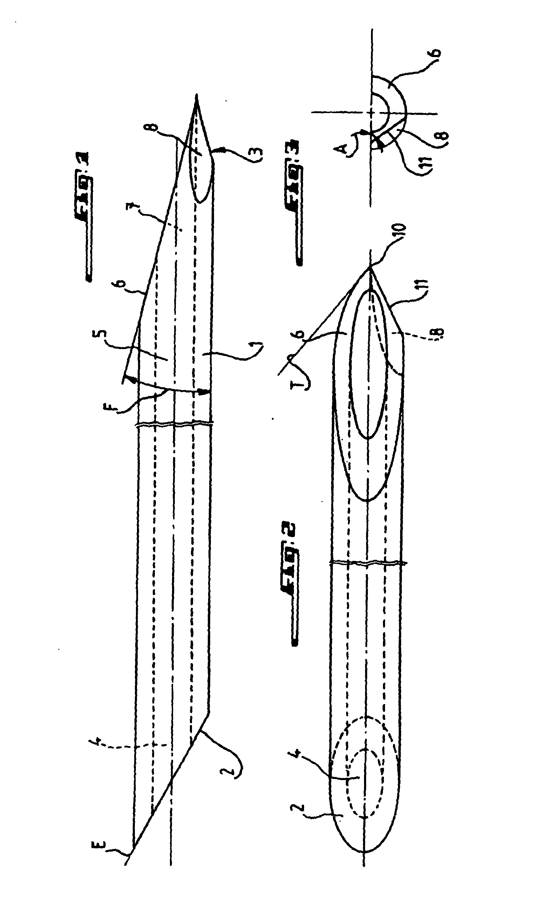

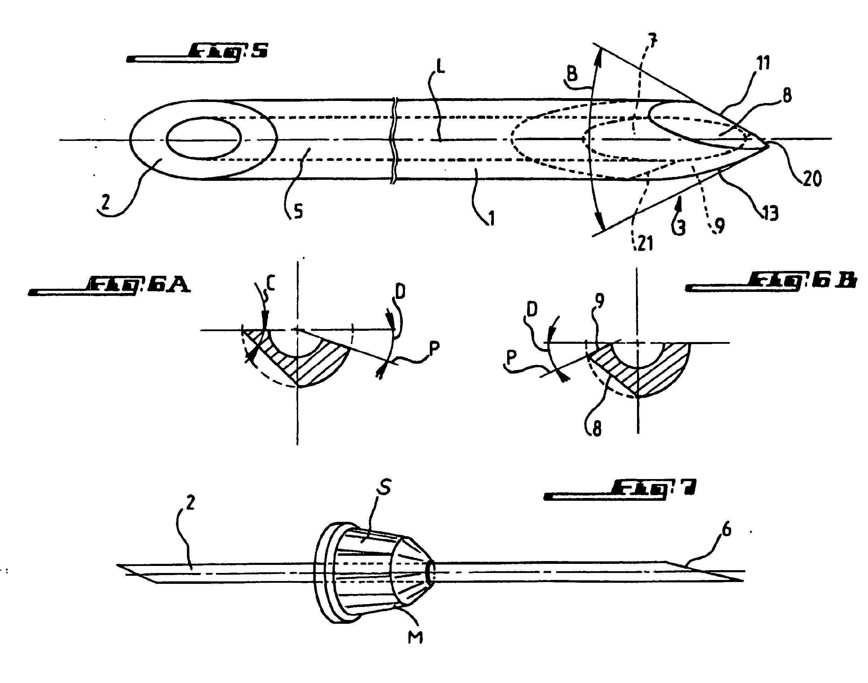

Une aiguille d'injection perforante selon l'invention, telle que représentée par exemple sur les figures 1 et 7, comprend un corps tubulaire 1 ayant deux extrémités opposées 2 et 3. La première extrémité 2 est destinée à être reliée, moyennant un support ou une embase S en plastique, à des moyens d'entraînement en rotation et à des moyens d'amenée de liquide, en l'occurrence un liquide pharmaceutique tel que, par exemple, un liquide anesthésique ou un liquide de traitement dermatologique. Le liquide est introduit dans l'aiguille d'injection par une entrée 4 d'un canal 5 traversant l'aiguille sur toute sa longueur. L'orientation de ce biseau détermine le marquage de l'aiguille, il doit donc être parallèle au biseau principal décrit ci-après.

La seconde extrémité 3 de l'aiguille est pourvue d'un biseau principal 6 et d'une ouverture 7 du canal 5. La seconde extrémité 3 est destinée à pénétrer dans un tissu dur ou mou dans lequel le liquide doit être déposé. Alors que la première extrémité 2 est formée par un biseau court 2 incliné avec un angle E d'environ 20 à 25 degrés par rapport à un axe longitudinal L de l'aiguille, la seconde extrémité 3 comporte un biseau long, le biseau principal 6 de cette extrémité, incliné avec un angle F d'environ 10 à 20 degrés par rapport à l'axe longitudinal L de l'aiguille.

Selon un premier mode de réalisation de l'invention, représenté sur les figures 2 et 3, l'aiguille d'injection perforante est pourvue, à la seconde extrémité 3, d'un biseau secondaire 8 disposé en opposition par rapport au biseau principal 6 et formant avec celui-ci un bord incisant 11 et une pointe 10 de l'aiguille. Le biseau principal 6 et le biseau secondaire 8 enferment entre eux d'une part un angle de coupe A de l'ordre de 40 degrés et d'autre part une pointe 10. Cette pointe peut être déplacée latéralement par rapport à l'axe pour faire varier l'angle de pointe B mesuré entre le bord incisant 11 et la tangente T au bord du biseau principal 6 adjacent au bord incisant 11 entre environ 30 à 40 degrés.

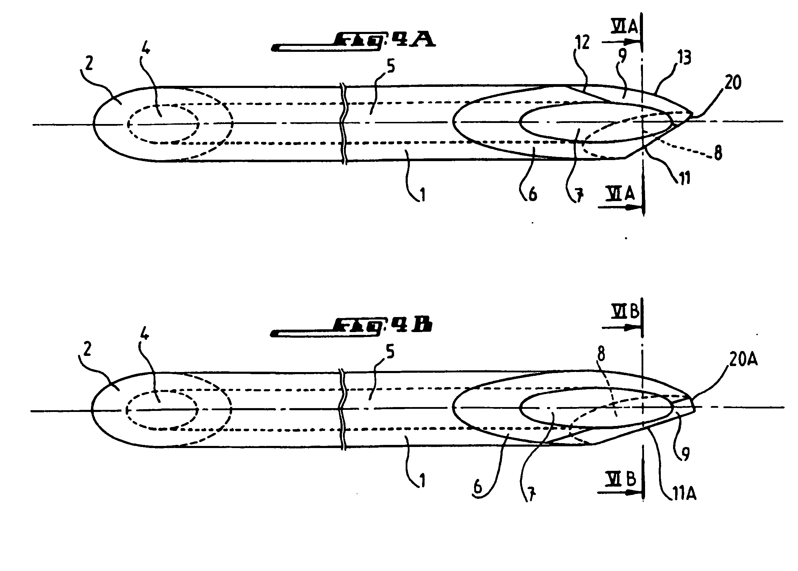

Selon un second mode de réalisation de l'invention, représenté sur les figures 4A, 5 et 6A, l'aiguille d'injection perforante est pourvue, à la seconde extrémité 3, de deux biseaux secondaires, un premier biseau secondaire 8 et un second biseau secondaire 9. Le premier biseau secondaire 8 est disposé en opposition par rapport au biseau principal 6 et forme avec celui-ci un bord incisant 11. Le second biseau secondaire 9 est disposé adjacent au biseau principal 6, sur un plan P incliné par un angle D de 20 à 35 degrés par rapport au biseau principal 6, et forme avec le biseau principal 6 une arête 12 et avec une partie du pourtour du corps tubulaire 1 opposée au biseau principal 6, un bord auxiliaire de coupe 13. Le biseau principal 6 et les deux biseaux secondaires 8, 9 forment ensemble une pointe de l'aiguille sous la forme d'une lame plate 20 résultant de l'intersection des deux biseaux secondaires 8, 9 à l'image d'une lame de bistouri.

Par ailleurs, les biseaux secondaires 8, 9 sont orientés de manière qu'un seul de ces deux biseaux, selon le mode de réalisation représenté, le biseau secondaire 8, forme avec le biseau principal 6 un bord incisant 11 bien sur l'ensemble de ces deux biseaux peut être inversé par rapport à l'axe de l'aiguille, pour rendre possible une rotation inverse.

Toutefois, en variante, il est également possible que le second biseau secondaire 9 soit disposé sur le bord opposé du biseau principal 6 et que ce soit lui qui forme avec le premier biseau secondaire 8 un bord incisant 11A et une lame 20A (Fig. 4B). Selon cette variante, le plan P est alors disposé de l'autre côté de l'axe L (Fig. 6B).

Le premier et le second biseaux secondaires 8, 9 forment entre eux un angle de pointe B correspondant à celui du premier mode de réalisation et qui est donc de l'ordre de 35 à 45 degrés. Les deux biseaux secondaires 8, 9 sont par ailleurs disposés de manière que le biseau secondaire 8 ou 9 qui forme avec le biseau principal 6 le bord incisant 11, forme avec le biseau principal 6 un angle C de l'ordre de 40 degrés.

Les deux biseaux secondaires 8, 9 sont par ailleurs disposés de manière asymétrique par rapport à un plan médian de l'aiguille passant par un axe longitudinal L de l'aiguille, ce plan médian s'élevant perpendiculairement par rapport au plan du dessin. Ainsi, les deux biseaux secondaires 8, 9 sont disposés de manière qu'ils se rejoignent uniquement pour former la pointe 20 sous la forme d'une lame plate.

La capacité de perforation de l'aiguille de l'invention ne dépendant pas seulement de la disposition des biseaux secondaires 8, 9 par rapport au biseau principal 6, il est intéressant de rappeler que le biseau principal 6 forme avec l'axe longitudinal L du corps tubulaire 1 de l'aiguille, dans chacun des deux modes de réalisation décrits, un angle D de l'ordre de 10 à 20 degrés.

Pour faciliter le positionnement de l'aiguille d'injection selon l'invention dans des moyens d'entraînement en rotation ou dans un porte-aiguille réunissant la fonction d'entraînement et la fonction amenée de liquide, l'aiguille selon l'invention est pourvue d'un marquage de positionnement M réalisé sous la forme d'un marquage apporté sur l'embase S en plastique ou en métal. Le marquage M se situe sur le dos de l'aiguille par rapport au biseau principal 6. Ceci permet au praticien de savoir que le biseau principal 6 de l'aiguille est bien parallèle à la muqueuse.

## Revendications

1. Aiguille d'injection perforante pour l'injection d'un produit pharmaceutique dans un corps humain ou animal, l'aiguille comprenant un corps tubulaire (1) ayant deux extrémités opposées (2, 3), une première extrémité (2) destinée à être reliée à des moyens d'entraînement en rotation et à des moyens d'amenée de produit et une seconde extrémité (3) destinée à pénétrer dans le corps humain ou animal et pourvue d'un biseau principal (6) et d'au moins un biseau secondaire (8),
**caractérisée en ce que** la seconde extrémité (3) de l'aiguille est pourvue, en opposition par rapport au biseau principal (6), d'un seul biseau secondaire (8) formant avec le biseau principal (6) un seul bord incisant (11) et une pointe (10, 20) de l'aiguille.

2. Aiguille selon la revendication 1, **caractérisée en ce que** le biseau principal (6) et le biseau secondaire (8) enferment entre eux un angle de coupe (A) de l'ordre de 40 degrés.

3. Aiguille selon la revendication 1 ou 2, **caractérisée en ce que** le biseau principal (6) et le biseau secondaire (8) enferment entre eux un angle de pointe (B) de l'ordre de 30 à 40 degrés.

4. Aiguille selon l'une quelconque des revendications 1 à 3, **caractérisée en ce que** le biseau principal (6) et le biseau secondaire (8) forment ensemble une pointe pointue (10) de l'aiguille.

5. Aiguille selon l'une quelconque des revendications 1 à 3, **caractérisée en ce que** la seconde extrémité (3) de l'aiguille est pourvue d'un premier biseau secondaire (8) disposé en opposition par rapport au biseau principal (6) et formant avec celui-ci un bord incisant (11), et d'un second biseau secondaire (9) disposé en opposition au premier biseau secondaire (8) et adjacent au biseau principal (6), sur un plan incliné (P) par rapport au biseau principal (6), et formant avec le biseau principal (6) une arête (12) et avec une partie du pourtour du corps tubulaire (1) opposée au biseau principal (6), un bord auxiliaire de coupe (13), le biseau principal (6) et les deux biseaux secondaires (8, 9) formant ensemble une pointe (20) de l'aiguille sous la forme d'une lame plate.

6. Aiguille selon la revendication 5, **caractérisée en ce que** le second biseau secondaire (9) est incliné par rapport au biseau principal (6) d'un angle d'inclinaison (D) de l'ordre de 35 degrés.

7. Aiguille selon la revendication 5 ou 6, **caractérisée en ce que** le bord incisant (11) et le bord auxiliaire de coupe (13) enferment entre eux un angle de pointe (B) de l'ordre de 35 à 45 degrés.

8. Aiguille selon l'une quelconque des revendications 5 à 7, **caractérisé en ce que** les deux biseaux secondaires (8, 9) sont disposés de manière asymétrique par rapport à un plan médian de l'aiguille passant par un axe longitudinal (L) de l'aiguille.

9. Aiguille selon l'une quelconque des revendications 1 à 8, **caractérisée en ce que** le biseau principal (6) forme avec un axe longitudinal (L) du corps tubulaire un angle (F) de l'ordre de 10 à 20 degrés.

10. Aiguille selon l'une quelconque des revendications 1 à 9, **caractérisée en ce qu**'elle est pourvue d'un marquage de positionnement (M) disposé, par rapport à un axe longitudinal (L) de l'aiguille, opposé au biseau principal (6).

## Claims

1. Perforating injection needle for injecting a pharmaceutical product into a human or animal body, said needle comprising a tubular body (1) having two opposite ends (2, 3), a first end (2) designed to be connected to rotational driving means and to supply means for supplying a product, and a second end (3) designed to penetrate the human or animal body and provided with a main bevel (6) and at least one secondary bevel (8),
**characterized in that** the second end (3) of the needle is provided, opposite the main bevel (6), with a single secondary bevel (8) forming, with the main bevel (6), a single cutting edge (11) and a needle point (10, 20).

2. Needle according to claim 1, **characterized in that** the main bevel (6) and the secondary bevel (8) enclose between themselves a wedge angle (A) of approximately 40 degrees.

3. Needle according to claim 1 or 2, **characterized in that** the main bevel (6) and the secondary bevel (8) enclose between themselves a point angle (B) of approximately 30 to 40 degrees.

4. Needle according to any of claims 1 to 3, **characterized in that** the main bevel (6) and the secondary bevel (8) together form a sharp needle point (10).

5. Needle according to any of claims 1 to 3, **characterized in that** the second end (3) of the needle is provided with a first secondary bevel (8), opposite the main bevel (6), and forming a cutting edge (11) therewith, and a second secondary bevel (9) opposite the first secondary bevel (8) and adjacent to the main bevel (6), on a plane (P) which is inclined in relation to the main bevel (6), and forming with the main bevel (6) an edge (12) and forming, with a part of the perimeter of the tubular body (1) opposite the main bevel (6), an auxiliary cutting edge (13), the main bevel (6) and the two secondary bevels (8, 9) together forming a needle point (20) in the form of a flat blade.

6. Needle according to claim 5, **characterized in that** the second secondary bevel (9) is inclined in relation to the main bevel (6) by an angle of inclination (D) of approximately 35 degrees.

7. Needle according to claim 5 or 6, **characterized in that** the cutting edge (11) and the auxiliary cutting edge (13) enclose between themselves a point angle (B) of approximately 35 to 45 degrees.

8. Needle according to any of claims 5 to 7, **characterized in that** the two secondary bevels (8, 9) are arranged asymmetrically in relation to a median plane of the needle, passing through a longitudinal axis (L) of the needle.

9. Needle according to any of claims 1 to 8, **characterized in that** the main bevel (6) forms, with a longitudinal axis (L) of the tubular body, an angle (F) of approximately 10 to 20 degrees.

10. Needle according to any of claims 1 to 9, **characterized in that** it is provided with a positioning marking (M) opposite the main bevel (6) in relation to a longitudinal axis (L) of the needle.

## Patentansprüche

1. Perforierende Injektionsnadel zur Injektion eines pharmazeutischen Produkts in einen menschlichen oder Tierkörper, wobei die Nadel einen röhrenförmigen Körper (1) mit zwei gegenüberliegenden Enden (2, 3) umfasst, wobei ein erstes Ende (2) dazu bestimmt ist, mit rotierenden Antriebsmitteln und mit Mitteln zur Produktbeförderung verbunden zu werden, und ein zweites Ende (3) dazu bestimmt ist, in den menschlichen oder Tierkörper einzudringen und mit einer Hauptschrägkante (6) und mindestens einer sekundären Schrägkante (8) ausgestattet ist, **dadurch gekennzeichnet, dass** das zweite Ende (3) der Nadel gegenüberliegend im Verhältnis zur Hauptschrägkante (6) mit einer einzigen sekundären Schrägkante (8) ausgestattet ist, die mit der Hauptschrägkante (6) eine einzige Schneidkante (11) und eine Nadelspitze (10, 20) bildet.

2. Nadel nach Anspruch 1, **dadurch gekennzeichnet, dass** die Hauptschrägkante (6) und die sekundäre Schrägkante (8) zwischen sich einen Schneidwinkel (A) in der Größeordnung von 40 Grad einschließen.

3. Nadel nach Anspruch 1 oder 2, **dadurch gekennzeichnet, dass** die Hauptschrägkante (6) und die sekundäre Schrägkante (8) zwischen sich einen Spitzenwinkel (B) in der Größeordnung von 30 bis 40 Grad einschließen.

4. Nadel nach einem der Ansprüche 1 bis 3, **dadurch gekennzeichnet, dass** die Hauptschrägkante (6) und die sekundäre Schrägkante (8) gemeinsam eine spitze Spitze (10) der Nadel bilden.

5. Nadel nach einem der Ansprüche 1 bis 3, **dadurch gekennzeichnet, dass** das zweite Ende (3) der Nadel mit einer ersten sekundären Schrägkante (8) ausgestattet ist, die im Verhältnis zur Hauptschrägkante (6) entgegengesetzt angeordnet ist und mit dieser eine Schneidkante (11) bildet, und mit einer zweiten sekundären Schneidkante (9), die entgegengesetzt zur ersten sekundären Schrägkante (8) angeordnet ist und benachbart zur Hauptschrägkante (6) auf einer im Verhältnis zur Hauptschrägkante (6) geneigten Ebene (P) und mit der Hauptschrägkante (6) einen Grat (12) und mit einem der Hauptschrägkante (6) gegenüberliegendem Abschnitt des Umfangs des röhrenförmigen Körpers (1) einen Hilfsschneiderand (13) bildet, wobei die Hauptschrägkante (6) und die zwei sekundären Schneidkanten (8, 9) gemeinsam eine Nadelspitze (20) in Form einer flachen Klinge bilden.

6. Nadel nach Anspruch 5, **dadurch gekennzeichnet, dass** die zweite sekundäre Schrägkante (9) im Verhältnis zur Hauptschrägkante (6) in einem Neigungswinkel (D) in der Größe ordnung von 35 Grad geneigt ist.

7. Nadel nach Anspruch 5 oder 6, **dadurch gekennzeichnet, dass** die Schneidkante (11) und die Hilfsschneidkante (13) zwischen sich einen Spitzenwinkel (B) in der Größeordnung von 35 bis 45 Grad einschließen.

8. Nadel nach einem der Ansprüche 5 bis 7, **dadurch gekennzeichnet, dass** die zwei sekundären Schneidkanten (8, 9) asymmetrisch im Verhältnis zu einer Mittelebene der Nadel angeordnet sind, die durch eine Längsachse (L) der Nadel verläuft.

9. Nadel nach einem der Ansprüche 1 bis 8, **dadurch gekennzeichnet, dass** die Hauptschrägkante (6) mit einer Längsachse (L) des röhrenförmigen Körpers einen Winkel (F) in der Größenordnung von 10 bis 20 Grad bildet.

10. Nadel nach einem der Ansprüche 1 bis 9, **dadurch gekennzeichnet, dass** sie mit einer Positionierungsmarkierung (M) ausgestattet ist, die im Verhältnis zu einer Längsachse (L) der Nadel der Hauptschrägkante (6) gegenüberliegend angeordnet ist.
